(19) Europäisches Patentamt
European Patent Office
Office européen des brevets

(11) Publication number: 0 016 444
A1

# (12) EUROPEAN PATENT APPLICATION

(21) Application number: 80101362.4

(22) Date of filing: 14.03.80

(51) Int. Cl.³: A 61 K 31/28
C 07 F 7/30

(30) Priority: 15.03.79 JP 30297/79

(43) Date of publication of application: 01.10.80 Bulletin 80/20

(84) Designated Contracting States: CH DE FR GB IT NL SE

(71) Applicant: Asai Germanium Research Institute
3-8- Mukohgaoka 2-chome
Bunkyo-ku Tokyo(JP)

(72) Inventor: Ishida, Nakao
1-5-40, Tsunogoroh
Sendai-shi Miyagi(JP)

(72) Inventor: Satoh, Hiroshi
1-26 Kami-Nakazato
Kata-ku Tokyo(JP)

(72) Inventor: Suzuki, Fujio
Oritate 01-3065 4-2 Moniwa-aza-Kobatayama
Sendai-shi Miyagi(JP)

(72) Inventor: Miyao, Kouhei
13-16 Nishikata 2-chome
Bunkyo-ku Tokyo(JP)

(74) Representative: Lorenz, Eduard Rechtsanwälte Eduard Lorenz- Bernhard Seidler- et al,
Margrit Seidler-Dipl.-Ing. Hans Karl Gossel- Dr. Ina Philipps-Dipl.-Chem. Rainer Wulf Widenmayerstrasse 23
D-8000 München 22(DE)

## (54) Method and agent for inducing an interferon.

(57) An agent for inducing an interferon represented by the following formula:

$(GeCH_2CH_2COX)_2O_3$

Wherein X stands for an -OH, $-NH_2$ or -O-Alkyl group.

EP 0 016 444 A1

./...

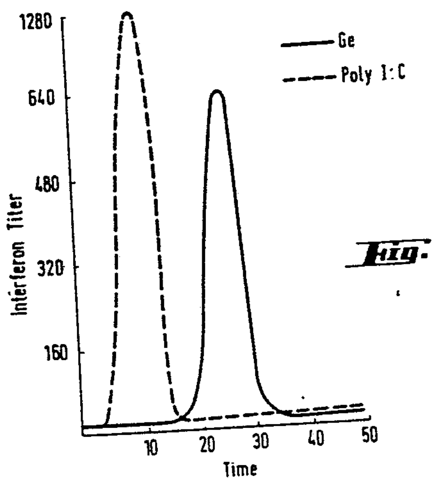
1280
640
480
320
160
Interferon Titer
Ge
Poly I:C
10
20
30
40
50
Time

Fig. 1

## DETAILED DESCRIPTION

In such state of the art, the inventors conducted screening with a view to finding an interferon inducing agent having low molecular weight and having low toxicity, and as a result, it was found that an organic germanium compound represented by the following formula:

$$(GeCH_2CH_2COX)_2O_3 \qquad (1)$$

Wherein X stands for an -OH, $-NH_2$ or -O-alkyl group, and is an interferon inducing agent satisfying the above reqirement.

Compounds represented by the above formula (1) can be prepared according to various methods. For example, a compound of the formula (1) in which X is an -OH group, i.e., carboxyethyl germanium sesquioxide, can be prepared by hydrolyzing cyanoethyltrichlorogermane with hydrochloric acid or sulfuric acid, or by reacting trichlorogermane with acrylic acid to form trichlorogermylpropionic acid and hydrolyzing the so formed trichlorogermylpropionic acid, or by chlorinating trichlorogermylpropionic acid to form trichlorogermylpropionyl chloride and hydrolyzing the so obtained trichlorogermylpropionyl chloride.

Furthermore, compounds represented by the above formula (1) can be prepared by reacting a trihalogenogermane represented by the following formula:

$$X_3GeH \qquad (2)$$

Wherein X stands for a halogen atom,

with chloropropionic acid or its derivative represented by the following formula:

$$ClCH_2CH_2COR \qquad (3)$$

Wherein R stands for an $-OH$, $-NH_2$ or $-O$-alkylene group, to form a compound represented by the following formula:

$$X_3GeCH_2CH_2COR \qquad (4)$$

Wherein X and R are as defined above, and hydrolyzing the so formed compound of the formula (4).

One of the characteristic features of the interferon inducing agent of the present invention is that the toxicity is very low. For example, in the case of carboxyethyl germanium sesquioxide, the $LD_{50}$ value is larger than 6300 mg/kg of the body weight of mice and is larger than 10 g/kg of the body weight of rats at the time of oral administration. At the time of intravenous infection, the $LD_{50}$ value is larger than a physically applicable maximum amount, i.e., 1000 mg/kg of the body weight, for either mice or rats. Furthermore, during the chronic toxicity test, no rat died in any of the groups in which the interferon inducing agent was orally administered over a period of 6 months at a dose of 30 mg, 300 mg or 3000mg per kg of the body weight per day, and no pathological disorder was observed. In the case where the interferon inducing agent was intravenously injected into dogs over a period of 6 months at a dose of 500 mg per kg of the body weight per day, no toxicity was found. Such low toxicity is seldom observed in the conventional interferon inducing agents.

The interferon induced by the agent of the present invention are proved to be Type II interferon, or so-called immune interferon, and it was also confirmed that this interferon is a factor to activate macrophage and the activated macrophage has strong cytotoxicity, that is, cell-kill activity against foreign cell such as cancer cells. So the agent of the present invention is said to be immuno modulater as well

as interferon inducing agent and used as chemotherapeutics.

The interferon inducing agent of the present invention will now be described in detail by reference to the following experiments, where the interferon inducing agent of the present invention is referred to as "Ge".

Experimental Example 1

Examination of the viral infection inhibitory activity to be induced by Ge was made. A 300 mg/kg dose of Ge was administered orally to a group of 30 DDI 6-week-old mice as the treated group. As the positive control group, 30 mice were administered intraperitoneally with a 0.5 mg/kg dose of polyinosinic acid polycytidylic acid (poly I:C). In addition, saline was administered to another 30 mice as a control group. From each group, the blood from 5 mice was taken at 1, 5, 10, 15, 20, 25, 30 and 50 hours after administration. The blood was subjected to centrifugation at 3,000 rpm for 15 minutes to separate the serum. The quantitative analysis of the viral infection inhibitory substance induced in the mouse sera was carried out in the following way. Three-day-old L-1D cells, grown in small tubes with Eagle's MEM supplemented 10% bovine serum (growing medium), were pretreated with the sera for 18-24 hours. Then cells were washed twice with the maintenance medium (Eagle's MEM + 2% bovine serum). They were then infected with vesicular stomatitis virus (VSV) at 100 $TCD_{50}$ (50% tissue culture infected dose)/ml. After 18-24 hours cultivation at 37°C. They were inspected by microscope in order to examine the inhibitory activity of viral infection.

Antiviral titer was expressed by the reciprocals of the highest dilution of the serum which significantly inhibits the viral cytopathic

effect (CPE). As shown in Fig. 1, viral infection inhibitory activity was detected in the serum of mice 25 hours after Ge administration.

Experimental Example 2

To determine the dose-dependent effect of Ge on the inducing activity of viral infection inhibitory activity, this test was carried out in the same manner as described in Experimental Example 1, except that the administered doses of Ge were changed.

The result obtained are shown in Fig. 2. The peak titer of viral infection inhibitory activity was detected 25 hours after Ge administration. From the figure, it can be seen that the inducing activity of Ge for a viral inhibitor depends on the amount administered and a high administered dose results in induction of a high titer of the viral inhibitor in circulation.

Experimental Example 3

In order to examine the biological properties of the viral infection inhibitory activity, the following experiment was conducted. Twenty mice, each weighing 20-22 g, and three human volunteers were given Ge orally at doses of 300 (mouse) and 75 (human) mg/kg. Twenty five hours (mouse) or 30 hours (human) after the administration, blood was taken and subjected to centrifugal separation for 15 minutes at 3,000 rpm. The two kinds of serum were thus prepared. As to the serum, the viral infection inhibitory effect was investigated using the primary culture cells of human embryonic lung and L-1D cells in the same manner as described in Experimental Example 1.

The result is shown in table 1. As indicated in the table, the viral infection inhibitory substance induced by Ge in mice showed resistance to the viral infection only in the L-1D cells and showed

no inhibitory activity to the viral infection in primary cultures of human embryonic lung and chicken embryonic cells. Similarly, the viral infection inhibitory substance induced in human volunteers showed viral resistant activity only in human cells. Accordingly, it can be said that the viral infection inhibitory substance induced by Ge shows species specificity.

Table 1.

| Interferon group | Interferon Titer/ 1 ml serum | | |
|---|---|---|---|
| | L-1D cell | Human embryonic lung cell | Chicken embryonic cell |
| Mouse | 640 | < 20 | < 20 |
| Human | < 20 | 160 | < 20 |

Experimental Example 4

In order to examine the physico chemical properties of the viral infection inhibitory substance induced in mouse serum by Ge in accordance with the method shown in Experimental Example 1 and that induced in mouse serum by poly I:C in the positive control group, the following experiments were conducted. One group was heated for one hour at 56°C. Another group was adjusted to pH 2.0 by dialyzing against a pH 2.0 buffer (1/100 M glycin-HCl buffer) and left for 18 hours at 4°C. Then the group was adjusted again to pH 7.2 by redialyzing against 1/100 M PBS. Still another group was treated by trypsin with a 1000 mcg/ml final concentration at 37°C for 3 hours. In the above test group, viral infection inhibitory activity was examined in line with Experimental Example 1.

The result is shown in table 2. As observed there, the viral

infection inhibitory substance induced in the mouse serum by Ge is unstable in thermal treatment of 56 C for 1 hour and to acidic treatment of pH 2.0, and its protein is susceptible to trypsin. Its properties are not similar to those of interferon induced by poly I:C excluded trypsin susceptibility.

As indicated there and in Experimental Examples 1 and 3, the viral infection inhibitory substance found in mouse serum induced by Ge was considered to be interferon. Especially, from the results of thermal and acidic treatments, this viral inhibitor induced by Ge in mouse serum is presumed to be immune (type II) interferon.

Table 2.

| Treatment | Interferon Titer/ 1 ml serum | |
|---|---|---|
| | Ge (Remaining rate of titer %) | Poly I:C (Remaining rate of titer %) |
| 56 C, heating 1 hr. | 20 ($<$ 3.1) | 640 (100) |
| pH 2.0, 4 C, standing for 18 hrs. | 20 ($<$ 3.1) | 640 (100) |
| Trypsin-treatment (1000 mcg/ml), 37°C, 3 hrs. | 20 ($<$ 3.1) | 20 ($<$ 3.1) |
| Non-treatment (kept in refrigerator) | 640 (100) | 640 (100) |

Experimental Example 5

It is known that interferon is able to convert a resting macrophage into a tumoricidal macrophage, called activated macrophage (A-MΘ), either in vivo or in vitro. Since Ge induced immune interferon in vivo, it is expected that A-MΘ will be induced in the Ge-administered animal.

Accordingly, the following tests were carried out to obtain the results shown in Table 3. Peritoneal exudate cells obtained from mice 72 hours after treatment with a 300 mg/kg dose of Ge were cultured for 20 minutes in a petri dish by using RPMI-1640 medium supplemented with 10% fetal calf serum. Adherent cells to the petri dish, obtained by washing with the same culture medium, were co-cultured with FM-3A cells as the target tumor cells at a ratio of 10:1, separately. FM-3A cells were cultured singly or with macrophages derived from normal mice, as the control. In each run, the growth of FM-3A cells was traced. A determination of the number of FM-3A cells was carried out by the dye exclusion test using Trypan-Blue. As is seen from the results shown in Table 3, the growth of FM-3A cells 48 hours after cultivation was prominently inhibited only in the case of co-culturing with the macrophages derived from the Ge-administered mice. Thus, it has been confirmed that Ge induces A-MΘ in the living bodies of mice.

Table 3.

| Culture system | Cell number after 48 hrs cultivation | Growth inhibitory rats (%) |
|---|---|---|
| FM-3A alone | $4.8 \times 10^5$ | 0 |
| FM-3A MΘ derived from normal mice | $4.4 \times 10^5$ | 8 |
| FM-3A MΘ derived from Ge treated mice | $2.5 \times 10^5$ | 48 |

Experimental Example 6

Ge was administered orally at a dose of 300 mg/kg to 50 male DDI mice (6-weeks-old). Twenty five hours later, they were sacrificed and serum was collected as an interferon sample induced by Ge in mice. Eighteen ml of the serum was obtained.

The anti-tumor activity ot the serum mentioned above as the Ge-induced interferon specimen was examined in tumor bearing mice. Serum collected from mice treated with physiological saline was used as a control. $4 \times 10^3$ cells/mouse of MEP-II tumor cells which were induced by 3-methylcholanthrene in DDI mice were inoculated in the peritoneal cavity of 40 mice.

Ten mice out of the 40 were given the serum interferon derived from Ge-treated mice. Another group of ten mice was given the serum obtained from the saline treated mice and all of the rest were treated with physiological saline. Administration of sera was started 24 hours after transplantation and was conducted once daily for 5 days at a dose of 0.2 ml per day.

As is seen from the results shown in Table 4, a significant survival effect was observed only in the Ge-induced interferon-administered group. Thus, it has been confirmed that Ge-induced interferon is anti-tumor active.

Table 4.

| Treatment | Number of treated mice | Survival days | Survival rate (%) |
|---|---|---|---|
| Serum IF derived from Ge treated mouse | 10 | >95.5 | 6/10 (60) |
| Serum derived from normal mouse | 10 | 28.0 | 0/10 (0) |
| Physiological saline | 20 | 27.2 | 0/20 (0) |

Experimental Example 7

An anti-tumor activity test of peritoneal exudate cells derived from mice treated with Ge at a dose of 300 mg/kg was conducted

on Ehrlich ascites tumor bearing mice.

$1 \times 10^4$ cells/mouse of Ehrlich ascites tumor cells were transplanted into the peritoneal cavity of DDI mice. Twenty four hours later, these mice were intraperitoneally given $5 \times 10^5$ peritoneal exudate cells obtained from mice treated with Ge once daily for 6 consecutive days. Peritoneal exudate cells derived from saline injected mice were given to tumor bearing mice as a control.

The survival rate of the three group was compared. As shown in Fig. 3, it was confirmed that the macrophage activated by Ge treatment exhibited anti-tumor activity.

Experimental Example 8

$4 \times 10^3$ MEP-II tumor cells were transplanted intraperitoneally in DDI mice as described above. Twenty four hours later, Ge was orally administered once daily for 7 consecutive days at a daily dosage of 100 or 300 mg/kg to examine the anti-tumor effect. In a control group, 0.2 ml of physiological saline solution was orally administered once daily for 7 days. As is seen from the results shown in Table 5, both doses of Ge exhibited anti-tumor activity.

When the results obtained are taken into account with the results in the foregoing experiments, it can readily be seen that Ge induces the production of an interferon. The induced interferon converts a resting macrophage to an A-MØ which kills or injures tumor cells and mice are able to survive.

Table 5.

| Treatment | Number of mice | Mean survival day | Survival rate(%) |
|---|---|---|---|
| Ge 300 mg/kg | 20 | > 102.0 | 55 |
| Ge 100 mg/kg | 20 | > 98.8 | 35 |
| Physiological Saline 0.2 ml/head | 40 | 29.6 | 0 |

Experimental Example 9

Capsules of Ge were orally administered to eight healthy volunteers of 21 to 23 years old. The dose administered to one group (two men and two women) was 75 mg/kg and the administrated amount to the other group (two men and two women) was 25 mg/kg. Serum samples were collected at predetermined intervals, the interferon titers were determined, and the results shown in Fig. 4 and 5 were obtained. In the 75 mg/kg-dose group, significant production of interferon was observed in each case. In the 25 mg/kg-dose group, production of interferon was observed although the level was low. This suggest that Ge induces A-MΦ in humans as well as in mice.

As should be apparent from the foregoing results, the interferon inducing agent of the present invention produces an immune interferon which exerts anti-tumor activity by activating macrophage. Furthermore, this interferon inducing agent has an extremely low toxicity when compared to known interferon inducing agents. Therefore, the interferon inducing agent of the present invention should be a very valuable carcinostatic agent.

## BRIEF DESCRIPTION OF THE DRAWINGS

Fig. 1 is a graph illustrating the amount of interferon induced according to the lapse of time when Ge is administered to mice of DDI.

Fig. 2 is a graph illustrating the relationship between the amount of interferon induced and the amount of Ge administered.

Fig. 3 is a graph illustrating the effect of abdominal cavity macrophage activated by Ge on the survival rate of Ehrlich ascites tumor bearing mice.

Fig. 4 and 5 are graphs illustrating the amount of interferon induced according to the lapse of time when Ge is administered to men.

WHAT IS CLAIMED IS:

1. An agent for inducing an interferon represented by the following formula:

$$(GeCH_2CH_2COX)_2O_3$$

Wherein X stands for an $-OH$, $-NH_2$ or $-O$-alkyl group.

2. An agent as in claim 1, wherein said agent is used as a macrophage activator.

3. An agent as in claim 1, wherein said agent is used as an immuno modulator.

4. An agent as in claim 1, 2 and 3, wherein said agent is used as chemotherapeutics for virus infection disease and neoplasia.

5. An agent as in claim 4, wherein the preparation of said chemotherapeutics are capsules, tablets, injection, liquids, supositories and the like.

6. A method for increasing an interferon titer of a patient comprising an interferon increasing effective amount of the agent as defined in claim 1.

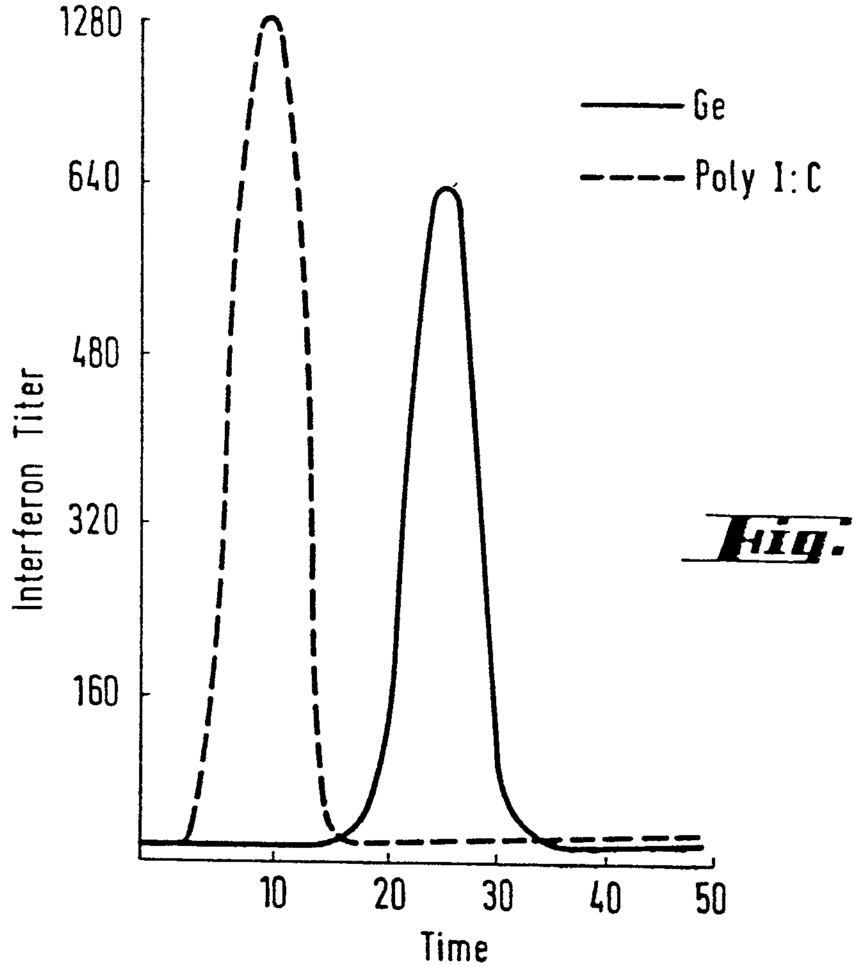
1280
640
480
320
160
Interferon Titer
Ge
Poly I:C
10
20
30
40
50
Time

Fig. 1

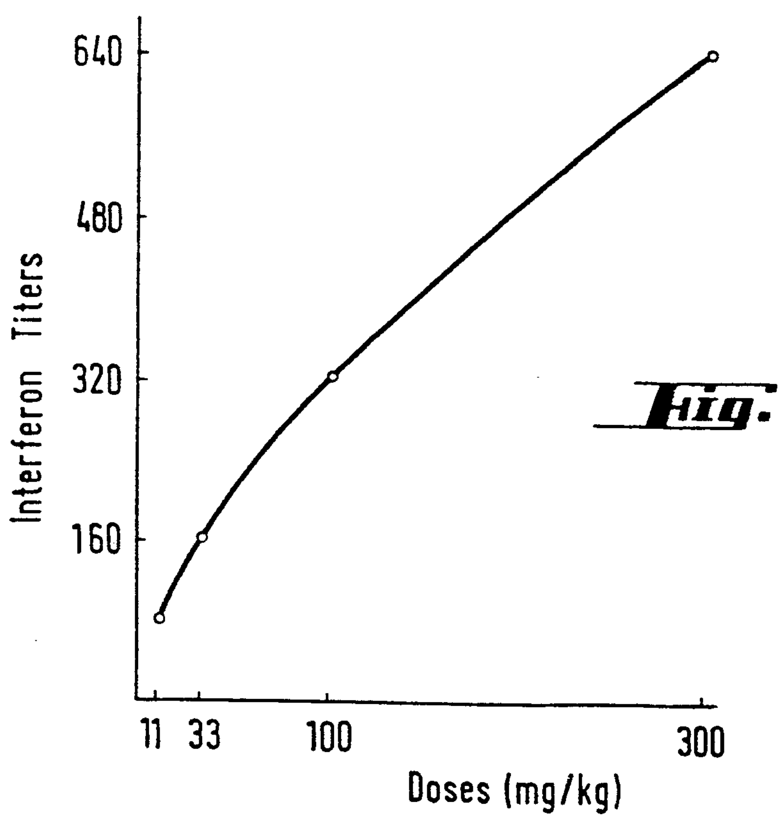
640
480
320
160
Interferon Titers
11
33
100
300
Doses (mg/kg)

Fig. 2

Fig. 3

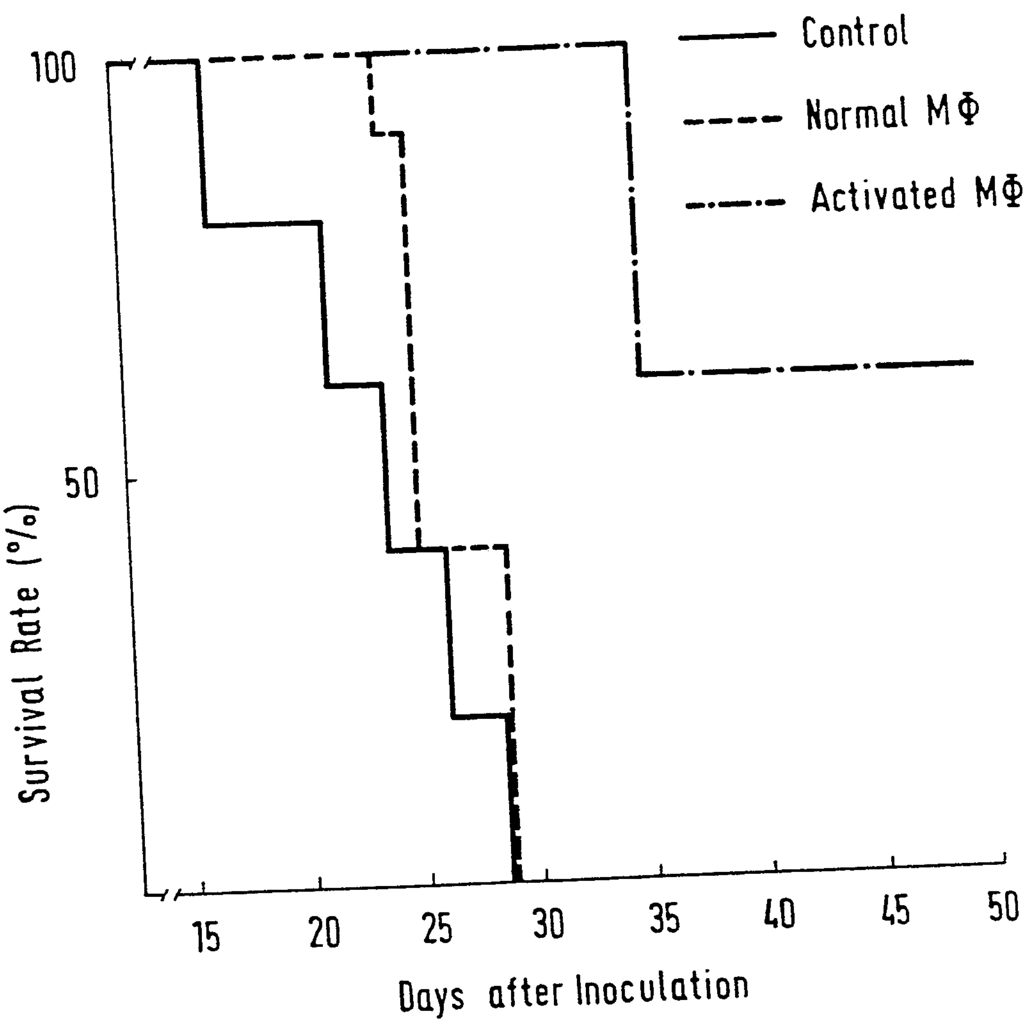

Control
Normal MΦ
Activated MΦ
100
50
Survival Rate (%)
15
20
25
30
35
40
45
50
Days after Inoculation

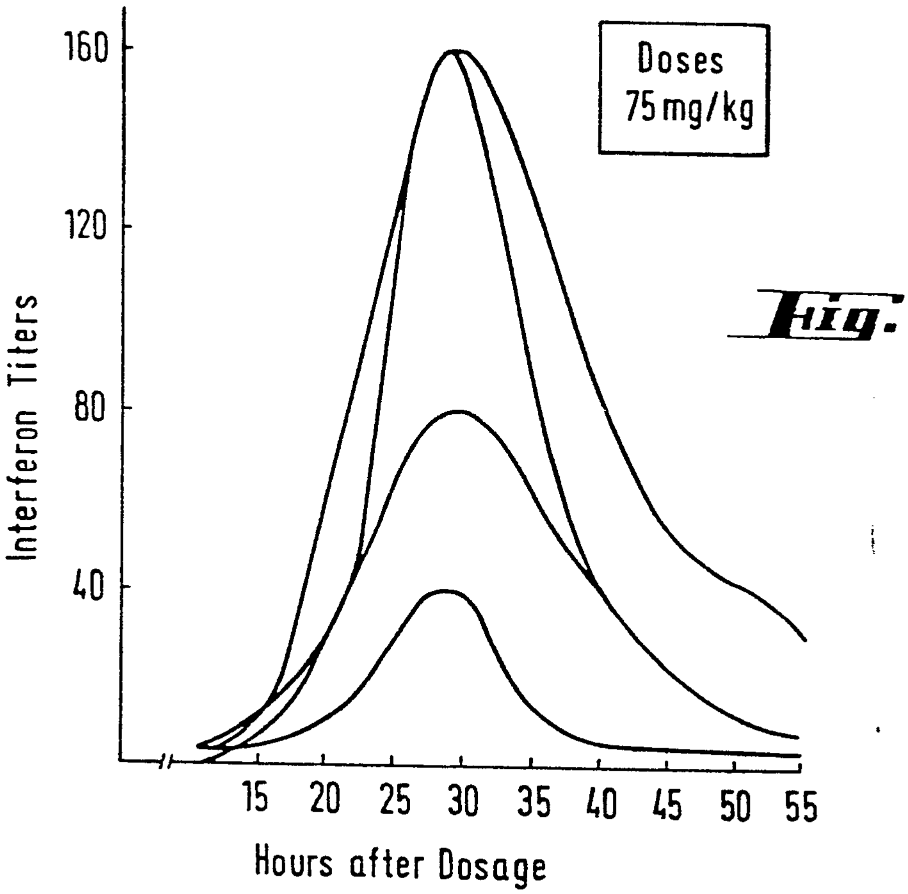
160
120
80
40
Interferon Titers
Doses
75 mg/kg
15 20 25 30 35 40 45 50 55
Hours after Dosage

Fig. 4

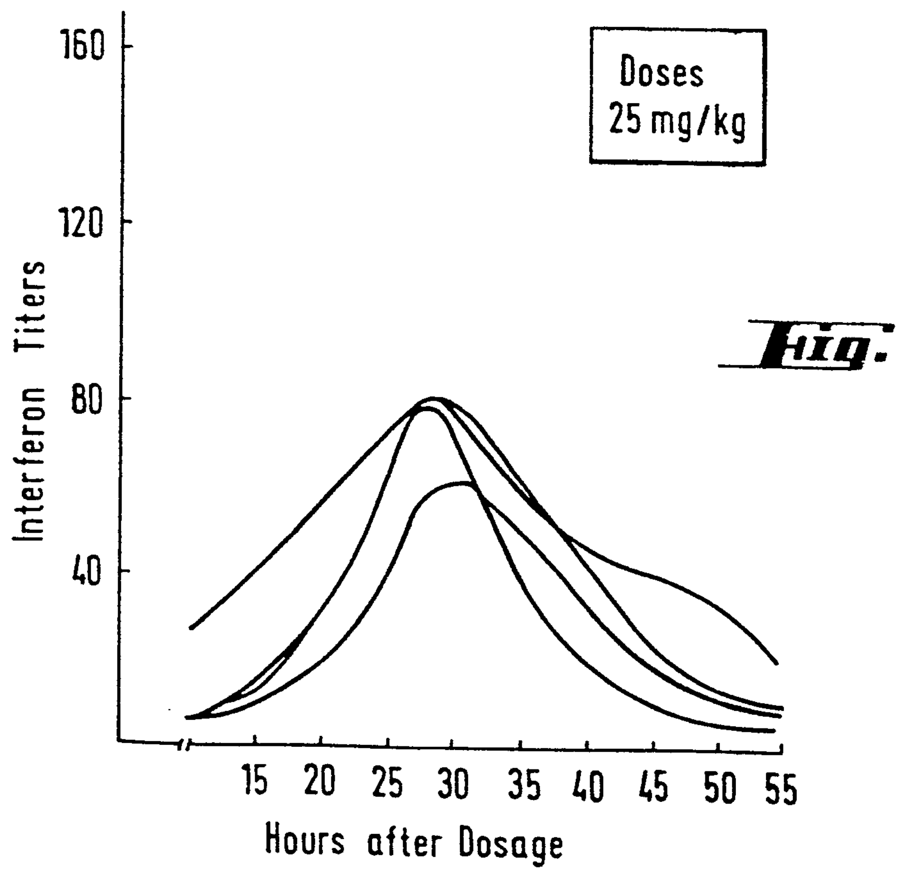
160
120
80
40
Interferon Titers
Doses
25 mg/kg
15 20 25 30 35 40 45 50 55
Hours after Dosage

Fig. 5

0016444

European Patent Office

# PARTIAL EUROPEAN SEARCH REPORT

which under Rule 45 of the European Patent Convention shall be considered, for the purposes of subsequent proceedings, as the European search report

Application number
EP 80 10 1362

## DOCUMENTS CONSIDERED TO BE RELEVANT

| Category | Citation of document with indication, where appropriate, of relevant passages | Relevant to claim | CLASSIFICATION OF THE APPLICATION (Int Cl.³) |
|---|---|---|---|
| X | US - A - 3 793 455 (KAZUHIKO ASAI)<br>* Column 1, lines 11-19 *<br>-- | 1-5 | A 61 K 31/28<br>C 07 F 7/30 |
| X | FR - A - 2 005 110 (DAIICHI YAKU-HIN SANGYO K.K.)<br>* Page 9, lines 1-17; page 9, line 39 - page 10, line 2 *<br>-- | 1-5 | |
| X | CHEMICAL ABSTRACTS, vol. 74, no. 23, 7th June 1971, page 491, no. 125852p<br>Columbus, Ohio, U.S.A.<br>& JP - A - 71 02964 (K. ASAHI et al.) 25-01-1971<br>* Abstract *<br>---- | 1-5 | TECHNICAL FIELDS SEARCHED (Int. Cl.³)<br>A 61 K 31/28<br>C 07 F 7/30 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5
Claims searched incompletely:
Claims not searched: 6
Reason for the limitation of the search: Method for treatment of the human or animal body by surgery or therapy (See article 52(4) of the European Patent Convention).

CATEGORY OF CITED DOCUMENTS
- X: particularly relevant
- A: technological background
- O: non-written disclosure
- P: intermediate document
- T: theory or principle underlying the invention
- E: conflicting application
- D: document cited in the application
- L: citation for other reasons
- &: member of the same patent family, corresponding document

| Place of search | Date of completion of the search | Examiner |
|---|---|---|
| The Hague | 17-06-1980 | SUTER |

EPO Form 1505.1 06.78